# EUROPEAN PATENT APPLICATION

(11) **EP 1 440 693 A1**
(43) Date of publication of application: **28.07.2004**
(21) Application number: 04001180.1
(22) Date of filing: 21.01.2004
(51) Int. Cl.: A61K 38/28

(54) **Medicament particularly for the treatment of diabetes mellitus**

(30) Priority: 23.01.2003 AT 852003
(71) Applicant: Dzien, Alexander, Dr., 6020 Innsbruck (AT); Lackner, Erich, Dr., 6020 Innsbruck (AT)
(72) Inventor: Dzien, Alexander, Dr., 6020 Innsbruck (AT); Lackner, Erich, Dr., 6020 Innsbruck (AT)
(74) Representative: Torggler, Paul N.

(57) **Zusammenfassung**

Medikament insbesondere zur Behandlung von Diabetes mellitus, wobei das Medikament eine Mischung aus einem ultrakurzwirksamen Insulin, vorzugsweise Insulinanalogon, und einem kurzwirksamen Insulin, vorzugsweise Normalinsulin, aufweist.

## Description

Die vorliegende Erfindung betrifft ein Medikament insbesondere zur Behandlung von Diabetes mellitus.

Diabetes mellitus (Zuckerkrankheit) stellt immer noch eine therapeutische Herausforderung an die heutige Medizin dar. Das wichtigste therapeutische Prinzip ist, den Glucosestoffwechsel des Patienten so weit wie möglich dem Stoffwechsel einer gesunden Person gleichzustellen. Die Regulierung des Glucosestoffwechsels erfolgt hormonell. Das wichtigste Hormon in der Regulierung ist das Insulin. Das Insulin wird von der Bauchspeicheldrüse produziert und von ihr bedarfsmäßig je nach der Höhe des Blutglucosewertes an die Blutbahn abgegeben. Eine normal funktionierende Bauchspeicheldrüse ermöglicht einen nahezu gleichmäßigen Verlauf des Blutzuckers (zwischen ca. 80-120 mg/dl) im Laufe des Tages. Diese "normale" Führung des Glucosestoffwechsels ist unerlässlich, um die Folgekrankheiten des Diabetes mellitus zu verhindern. Um die hohe Variabilität der Bauchspeicheldrüsenproduktion nachahmen zu können, wurden verschiedene Insuline entwickelt.

Die derzeit verwendeten Insuline werden nach folgenden Wirkkriterien charakterisiert:
- Wirkdauer: Das ist die Zeit, in der das Insulin im Körper nachweisbar ist.
- Wirkungsbeginn: Das ist die Zeitspanne, welche zwischen dem vom Zeitpunkt der Insulininjektion und dem Wirkungsbeginn vergeht.
- Dauer der maximalen Wirkung: Das ist die Zeit des Beginns der Maximalwirkung bis zum Ende der maximalen Wirkung.

Unter der Berücksichtigung dieser Parameter können folgende Arten von Insulin unterschieden werden:
1. ultrakurzwirksame Insuline (Insulinaloga, Monomere)
2. kurzwirksame Insuline (Normalinsuline)
3. mittellangwirksame Insuline (Isophan-Insuline)
4. langwirksame Insuline und Insulinanaloga

Um eine Verbesserung des Stoffwechsels zu erreichen, wurden bisher die Insuline auch miteinander vermischt. Hier finden sich vor allem Mischungen aus ultrakurzwirksamen und mittelwirksamen Insulinen sowie aus kurzwirksamen und mittelwirksamen Insulinen. Die bisherige Kontrolle des Therapieerfolges wurde entsprechend den internationalen Empfehlungen mittels Laborbefunden sowie eigenen Aufzeichnungen der Patienten durchgeführt.

Eine kontinuierliche Blutzuckermessung (BZ) wird seit neuestem durch Glucosesensoren (z.B. von der Firma Minin Med) über z.B. 72 Stunden ermöglicht.

Vergleiche der Langzeitmessungen mit dem Verlauf des Blutzuckerprofils einer gesunden Person zeigen oft neben den häufigsten Problemen der nächtlichen Stoffwechselführung ein Problem mit der Behandlung der prandialen (prandial = das Essen betreffend) Phasen (Reaktion des Glucosestoffwechsels auf die eingenommene Mahlzeit).

Hier zeigt sich häufig unabhängig von dem relativ guten 1 ½ bis 2 Stunden postprandialen Wert (nach dem Essen) ein im Vergleich zur Normalperson inadäquater Blutzuckeranstieg. Dieser wird teilweise durch eine fraktionierte Insulingabe des prandialen Insulins hervorgerufen, wobei diese fraktionierte Gabe durch eine nochmalige Injektion nach Beendigung der Mahlzeit erreicht wird.

Trotz der zahlreichen Versuche mit den unterschiedlichsten Wirkstoffen und Mischungen wurde bei allen beim Stand der Technik bekannten Medikamenten noch keine optimale Anpassung des Verlaufs des Blutzuckerprofils einer an Diabetes mellitus erkrankten Person an den einer gesunden Person erreicht.

Aufgabe der Erfindung ist es daher, ein Medikament zur Verfügung zu stellen, durch das eine gegenüber dem Stand der Technik weiter verbesserte Anpassung des Verlaufs des Blutzuckerprofils bei an Diabetes mellitus erkrankten Personen an den normalen Verlauf des Blutzuckerprofils von gesunden Personen erreicht wird.

Dies wird erfindungsgemäß dadurch erreicht, dass das Medikament eine Mischung aus einem ultrakurzwirksamen Insulin, vorzugsweise Insulinanalogon, und einem kurzwirksamen Insulin, vorzugsweise Normalinsulin, aufweist.

Hierdurch wird eine Verbesserung des prandialen Blutzuckerprofils erreicht, wobei das Insulin besser an die Qualität und Quantität der Nahrung angepasst ist. Insgesamt wird durch das erfindungsgemäße Medikament eine sehr gute Anpassung des Verlaufs des Blutzuckerprofils einer an Diabetes mellitus erkrankten Person an den Verlauf des Blutzuckerprofils bei einer gesunden Person erreicht. Hierbei kann für das erfindungsgemäße Medikament als ultrakurzwirksames Insulin z.B. das Insulin Lispro oder das Insulin Aspart verwendet werden. Als kurzwirksames Insulin können erfindungsgemäß z.B. das Insulin Actrapid der Fa. Novo-Nordisk, das Insulin Rapid der Fa. Aventis oder das Insulin Normal der Fa. Lilly verwendet werden.

Besonders günstige Ausführungsvarianten sehen bei der Mischung vor, dass das ultrakurzwirksame Insulin nach 0 min (Minuten) bis 15 min, vorzugsweise nach 0 min bis 10 min zu wirken beginnt und dass das ultrakurzwirksame Insulin eine Wirkdauer von 1 bis 3 Stunden, vorzugsweise von 2 Stunden bis 3 Stunden, aufweist. Darüber hinaus ist es günstig, wenn die Dauer der maximalen Wirkung des ultrakurzwirksamen Insulins nach 40 min bis 80 min, vorzugsweise nach 55 min bis 65 min, einsetzt.

Bezüglich des für die Mischung verwendeten kurzwirksamen Insulins sieht eine günstige Variante vor, dass dieses nach 10 min bis 40 min, vorzugsweise nach 15 min bis 30 min, zu wirken beginnt. Hierbei ist es wiederum günstig, dass das kurzwirksame Insulin eine Wirkdauer von 3 bis 9 Stunden, vorzugsweise von 4 Stunden bis 8 Stunden, aufweist und dass die Dauer der maximalen Wirkung des kurzwirksamen Insulins nach 100 min bis 180 min, vorzugsweise nach 120 min bis 150 min, einsetzt.

Günstige Ausführungsformen sehen darüber hinaus vor, dass das Mischungsverhältnis zwischen dem ultrakurzwirksamen und dem kurzwirksamen Insulin einem Verhältnis von 7 : 3 entspricht. Alternativ hierzu kann auch vorgesehen sein, dass das ultrakurzwirksame Insulin und/oder das kurzwirksame Insulin mindestens je 30 % und höchstens je 70 % Gewichtsanteil aufweist(en).

In einer besonders günstigen Ausführungsvariante ist vorgesehen, dass das Medikament zur Behandlung von Diabetes mellitus ausschließlich eine Mischung aus einem ultrakurzwirksamen Insulin und einem kurzwirksamen Insulin aufweist. In dieser Ausführungsvariante ist jedoch nicht ausgeschlossen, dass das Medikament noch mit anderen nicht der Behandlung von Diabetes mellitus dienenden Wirkstoffen kombiniert wird.

Allgemein steht bei der, mit den angeführten Ausführungsvarianten des erfindungsgemäßen Medikamentes durchgeführten, Therapie nicht die Struktur der einzelnen zu mischenden Insuline im Vordergrund, sondern die fixe oder variable Kombination der Eigenschaften der beteiligten Insulintypen. Ein Ausführungsbeispiel sieht hierbei die Mischung der folgenden zwei Insulintypen vor:
1. Ulktrakurzwirksames Insulin-Analogon B28 LysB29Pro und B28Asp, chrakterisiert durch: Wirkungseintritt nach 0 bis 15 min, dem Eintritt der maximalen Wirkdauer nach ca. 60 min und einer Wirkdauer von 2 bis 3 Stunden.
2. Kurzwirksames Normalinsulin, charakterisiert durch: Wirkungseintritt 15 bis 30 min nach der Injektion, dem Eintritt der maximalen Wirkdauer nach ca. 120 bis 150 min und einer Wirkdauer von 4 bis 8 Stunden.

In der beigelegten Figur ist die Wirkungsdauer und die Intensität eines Ausführungsbeispiels des erfindungsgemäßen Medikamentes mit der Wirkungsdauer und Intensität von herkömmlichem Analoginsulin und Normalinsulin verglichen. Durch die erfindungsgemäße Kombination verschiedener Insulintypen wird gleichzeitig ein schnelles Ansteigen und eine hohe Intensität der Wirkung kurz nach der Einnahme wie auch die gewünschte Langzeitwirkung des Insulins erreicht. Dies kann - wie dargestellt - von herkömmlichem Analoginsulin und herkömmlichem Normalinsulin nicht gewährleistet werden.

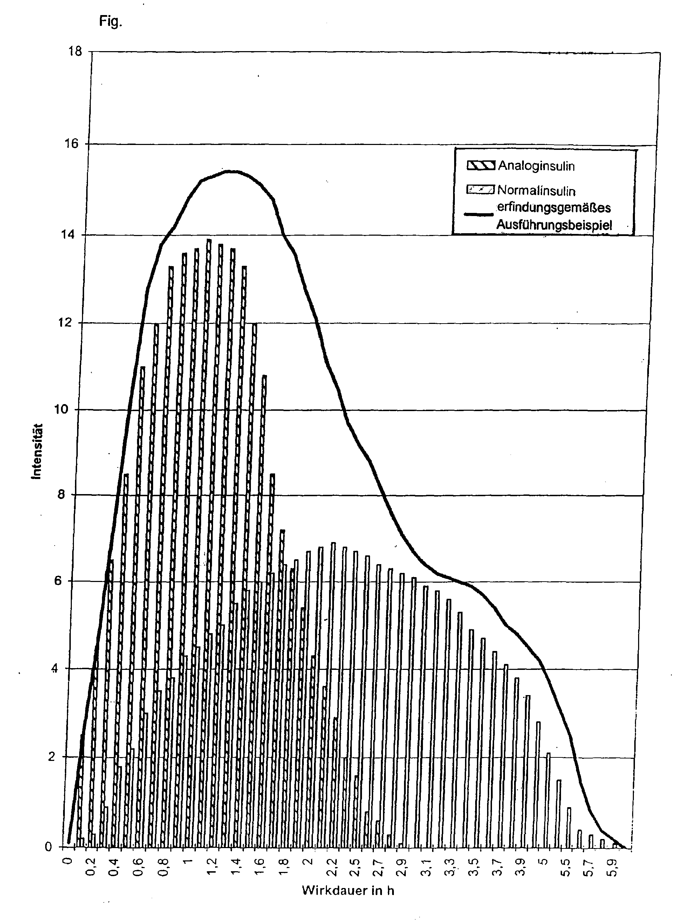

## Patentansprüche

1. Medikament insbesondere zur Behandlung von Diabetes mellitus, **dadurch gekennzeichnet, dass** das Medikament eine Mischung aus einem ultrakurzwirksamen Insulin, vorzugsweise Insulinanalogon, und einem kurzwirksamen Insulin, vorzugsweise Normalinsulin, aufweist.

2. Medikament nach Anspruch 1, **dadurch gekennzeichnet, dass** das ultrakurzwirksame Insulin nach 0 min bis 15 min, vorzugsweise nach 0 min bis 10 min, zu wirken beginnt.

3. Medikament nach einem der Ansprüche 1 oder 2, **dadurch gekennzeichnet, dass** das ultrakurzwirksame Insulin eine Wirkdauer von 1 bis 3 Stunden, vorzugsweise von 2 Stunden bis 3 Stunden, aufweist.

4. Medikament nach einem der Ansprüche 1 bis 3, **dadurch gekennzeichnet, dass** die Dauer der Wirkung des ultrakurzwirksamen Insulins nach 40 min bis 80 min, vorzugsweise nach 55 min bis 65 min, einsetzt.

5. Medikament nach einem der Ansprüche 1 bis 4, **dadurch gekennzeichnet, dass** das kurzwirksame Insulin nach 10 min bis 40 min, vorzugsweise nach 15 min bis 30 min, zu wirken beginnt.

6. Medikament nach einem der Ansprüche 1 oder 5, **dadurch gekennzeichnet, dass** das kurzwirksame Insulin eine Wirkdauer von 3 bis 9 Stunden, vorzugsweise von 4 Stunden bis 8 Stunden, aufweist.

7. Medikament nach einem der Ansprüche 1 bis 6, **dadurch gekennzeichnet, dass** die Dauer der Wirkung des kurzwirksamen Insulins nach 100 min bis 180 min, vorzugsweise nach 120 min bis 150 min, einsetzt.

8. Medikament nach einem der Ansprüche 1 bis 7, **dadurch gekennzeichnet, dass** das Mischungsverhältnis zwischen ultrakurzwirksamen und kurzwirksamen Insulin einem Verhältnis von 7 : 3 entspricht.

9. Medikament nach einem der Ansprüche 1 bis 7, **dadurch gekennzeichnet, dass** das ultrakurzwirksame Insulin und/oder das kurzwirksame Insulin mindestens je 30 % und höchstens je 70 % Gewichtsanteil aufweist(en).

10. Medikament nach einem der Ansprüche 1 bis 9, **dadurch gekennzeichnet, dass** es zur Behandlung von Diabetes mellitus ausschließlich eine Mischung aus einem ultrakurzwirksamen Insulin und einem kurzwirksamen Insulin aufweist.
